# FASCICULE DE BREVET EUROPEEN

(11) **EP 0 558 361 B1**
(45) Date de publication et mention de la délivrance du brevet: **15.04.1998**
(21) Numéro de dépôt: 93400236.1
(22) Date de dépôt: 01.02.1993
(51) Int. Cl.: G01N 33/556

(54) **Hématies indicatrices, une méthode de préparation et leur utilisation**
Sensibilisierte Erythrozyten, ein Verfahren zu deren Herstellung, und deren Verwendung
Sensitized erythrocytes, a method of preparing the same and use thereof

(30) Priorité: 24.02.1992 FR 9202081
(43) Date de publication de la demande: 01.09.1993
(73) Titulaire: ASSOCIATION POUR L'ESSOR DE LA TRANSFUSION SANGUINE DANS LA REGION DU NORD, 59000 Lille (FR)
(72) Inventeur: Mannessier,née Wartell,Lucienne, F-59000 Lille (FR)
(74) Mandataire: Lhuillier, René

(56) Documents cités:
- EP-A- 0 363 510
- WO-A-79/00796
- GB-A- 2 117 514
- IMMUNOLOGY vol. 9, no. 2, Août 1965, OXFORD GB pages 161 - 175 A FAGRAEUS ET AL. 'Mixed haemadsorption: a mixed antiglobulin reaction applied to antigen on a glass surface'

## Description

L'invention se rapporte au domaine de l'immuno-hématologie et plus particulièrement à la mise en évidence de complexes antigènes-anticorps à l'aide d'anticorps ou de cellules phénotypées immobilisés sur un support solide, et d'hématies indicatrices.

L'invention concerne spécifiquement lesdites hématies indicatrices, sur lesquelles sont fixées des antiglobulines polyvalentes et, plus particulièrement, des anti-IgG et des anti-C₃d, et une méthode d'obtention desdites hématies indicatrices.

L'invention est plus particulièrement destinée au domaine de l'immuno-hématologie et de la sécurité transfusionnelle (groupage sanguin ABO-D, épreuve de compatibilité, phénotypage érythrocytaire, recherche des anticorps irréguliers...), mais elle peut également s'appliquer à la détection indirecte d'autres anticorps ou antigènes en rapport avec diverses maladies.

Les divers tests de groupage sanguin et de compatibilité transfusionnelle ont été mis au point en phase liquide. Bien qu'universellement utilisées dans les laboratoires d'immuno-hématologie, ces réactions d'agglutination en phase liquide comportent cependant un certain nombre d'inconvénients. Elles sont très subjectives, peu quantitatives et peu sensibles. L'autre inconvénient majeur est l'impossibilité de lecture, au point final objectif de la réaction.

C'est pourquoi plusieurs laboratoires ont cherché à mettre au point des tests plus simples et plus fiables ; les progrès les plus significatifs ont été apportés par les tests appelés "en phase solide" c'est-à-dire avec un des éléments (anticorps ou cellules phénotypées) fixé de manière stable à un support en matière plastique, généralement une microplaque (à 96 puits) du type de celles conçues pour les cultures cellulaires, et la révélation indirecte de la réaction antigène-anticorps par immuno-adhérence d'hématies.

Les dispositifs adaptés au remplissage et à la manipulation de ces microplaques de manière automatique sont couramment disponibles.

Les tests d'immuno-adhérence décrits récemment sont basés sur des procédés décrits pour la première fois dans les années 1950. Les premières approches dérivent des travaux de Coombs (Coombs R.R.A. and Bedford D. Vox Sang. 1955 ; 5, 11) qui utilise des réactions d'agglutination mixte, pour mettre en évidence des antigènes de groupe sanguin sur les plaquettes (Ashurst D.E. Bedford D. and Coombs R.R.A. Vox Sang. 1956 ; 1, 235).

Bien qu'initialement le développement de cette technique d'agglutination mixte ait été réalisé en phase liquide, plus tard elle fut exploitée comme une méthode en phase solide, en médecine légale (Coombs R.R.A. and Dodd B.E. Med. Sci. Law. 1961 ; 1, 359).

La découverte de l'hémadsorption par certains virus sur les cellules infectées a constitué une autre étape préalable (Hogman C. Vox Sang. 1959 ; 4, 12) à la réalisation de la première réaction en phase solide par Hogman, qui combinait l'agglutination mixte et l'hémadsorption (Hogman C. Vox Sang. 1959 ; 4, 319). Des cultures cellulaires sont sensibilisées par l'anti-A ou l'anti-B et l'adhérence des globules rouges indicateurs révèle la présence de l'antigène sur les cellules cultivées. Dans ce modèle, la cellule étudiée et la cellule indicatrice doivent partager le même antigène.

Dès 1961, Fagraeus et Espmarck combinent l'agglutination mixte et la réaction d'hémadsorption (Nature 1961 ; 190, 370 et Fagraeus A. Espmarck J.A. and Johnson J. Immunology 1965 ; 9, 161). Le nouvel aspect de cette technique est l'utilisation de globules rouges indicateurs sur lesquels on a fixé de l'antiglobuline. Une variante technique du "marquage" à l'antiglobuline fut développée par Coombs qui utilisa du chlorure de chrome pour fixer directement l'antiglobuline ou d'autres protéines sur les globules rouges (Coombs R.R.A. Assays utilizing red cells as markers. In "Immunology for the 80's". Voller A, Ed. MTP Press, Lancester 1981).

En 1967, Catt et Tregear (Science 1967 ; 158, 1570), font l'importante observation que les molécules d'anticorps peuvent s'adsorber sur du polystyrène ou du polypropylène sans perdre leur activité immunologique. Cette découverte a permis l'extension des tests en phase solide.

Enfin, la possibilité d'utiliser des monocouches de globules rouges en sérologie a été documentée par Rosenfield en 1978. Les hématies étant fixées sur du plastique à l'aide de fibrinogène et de poly-D-lysine (Rosenfield R.E. New technical approaches to red cell serology. XV Congr. Int. Soc. Blood Transfusion Paris 1978), la monocouche d'hématies-tests pouvait ensuite adsorber l'anticorps spécifique en solution (sérum). Puis l'anticorps fixé était détecté soit par le biais d'une hémolyse immune (après addition de sérum AB, source de complément). Après incubation et lavage, les hématies intactes restantes étaient lysées et le taux d'hémoglobine mesuré.

Une autre méthode consistait à ajouter de l'antiglobuline après avoir lysé les hématies sensibilisées. La quantification était réalisée après lyse des cellules indicatrices fixées, par mesure de l'absorbance de l'hémoglobine libérée.

En 1982, Moore décrit un autre test à l'antiglobuline en phase solide (Moore H.H. and Conradie J.D. Transfusion 1982 ; 22, 540). Après sensibilisation des hématies-tests par les sérums en phase liquide, les hématies sont lavées puis transférées dans une microplaque où a été fixée de l'antiglobuline humaine. Après centrifugation, des réactifs peroxydasiques sont ajoutés pour détecter les hématies sensibilisées.

La plupart des tests en phase solide dérivent des méthodes originales décrites ci-dessus. L'application de ces tests à la détermination des groupes sanguins ABO-D a été adaptée à partir des techniques de Mage (Mage M.G., MC Hugh L.L. and Rothstein T.L., J. immunol. Methods. 1977 ; 15, 47) et de Wysocki (Wysocki L.J. and Sabo V.L. Proc. Natl. Acad. Sci U.S.A. 1978 ; 75, 2844).

La contre-épreuve du groupe ABO est une modification de la technique de culture tissulaire de Hogman (1959, cité plus haut).

De même, la recherche des anticorps irréguliers est basée sur l'agglutination mixte de Hogman aussi, et l'hémadsorption mixte de Fagraeus (1961, 1965).

On trouve la description de tests pour le groupage sanguin en microplaque dans la demande de brevet EP-A-0 084 102 de la Société Biotest et le brevet US-A-4 608 246 de la Société Immucor.

Le matériel Biotest® impose d'effectuer plusieurs centrifugations aux différentes étapes du test ce qui ne représente pas une simplification par rapport aux tests traditionnels en tubes ; de plus la nécessité de transférer les hématies sensibilisées d'une première microplaque à la microplaque-test représente une source potentielle d'erreur de manipulation trop dangereuse pour le domaine des transfusions sanguines.

Le matériel Immucor® est d'une utilisation plus simple et plus fiable mais les hématies indicatrices ne portent que de l'antiglobuline anti-IgG. Ce test ne peut donc détecter ni les IgM ni les IgG faibles fixant le complément.

La Demanderesse a maintenant découvert qu'il était possible de réaliser un nouveau modèle qui permet également de détecter les IgM et le complément, tout en reposant sur les mêmes principes théoriques de réaction immunologique en phase solide et de révélation des complexes antigènes-anticorps par immuno-adhérence d'hématies indicatrices, en préparant des hématies indicatrices qui portent à la fois de l'antiglobuline anal-IgG et de l'anti-complément, plus particulièrement de l'anti-C₃d monoclonal. De manière surprenante, ces hématies indicatrices sont suffisamment stables pour être conditionnées, transportées et utilisées en laboratoire, à grande échelle. Ainsi il est possible d'effectuer la totalité des tests de compatibilité sanguine à l'aide d'un seul réactif (les hématies indicatrices) et d'une seule microplaque préalablement recouverte de la batterie d'anticorps et/ou d'hématies-tests ou de leurs membranes.

L'invention concerne également la méthode de préparation des hématies indicatrices portant l'anti-IgG et l'anti-C₃d. Celle-ci comprend 3 étapes : les 2 premières sont destinées à fixer les 2 marqueurs sur les hématies pour donner ce qu'on appelle des hématies "sensibilisées", la dernière est destinée à fixer les antiglobulines sur ces marqueurs.

Les hématies indicatrices proviennent d'un "pool" d'hématies O Rh positif DCcEe.

La première étape consiste à fixer le complément (on utilise un pool de sérum AB frais comme source de complément) sur les hématies, réaction qui est provoquée par l'addition de saccharose dans la solution de tampon à basse force ionique.

Le complément ainsi adsorbé est au stade C₃bi ; on peut provoquer son activation au stade C₃d par un traitement à la trypsine. (Cette étape n'est pas indispensable parce que l'antiglobuline anti-C₃d reconnaît aussi le fragment C₃bi).

Les hématies sensibilisées au C₃d ainsi obtenues sont ensuite mises en présence d'immunoglobulines de classe IgG dressées contre un marqueur antigénique des hématies. On utilise de préférence une immunoglobuline monoclonale pour obtenir une meilleure reproductibilité et, par exemple, on utilise de l'IgG anti-D.

Les hématies indicatrices sont donc sensibilisées au C₃d et aux IgG. La troisième étape consiste à fixer les antiglobulines correspondantes sur ces deux marqueurs, par immuno-adhérence. Les concentrations d'antiglobuline anti-IgG et anti-C₃d doivent être ajustées pour permettre la fixation des hématies indicatrices sur les complexes antigènes-anticorps dans la microplaque du test, mais pour ne pas permettre une agglutination des hématies indicatrices, entre elles.

L'utilisation d'anticorps monoclonaux permet une bonne standardisation des procédés de préparation et en assure la reproductibilité, alors qu'avec les anticorps d'origine humaine, la constante d'affinité peut varier de 1 à 10³ d'un donneur à l'autre.

La solution d'hématies indicatrices selon l'invention, préparées par cette méthode est prête à l'emploi, pour les tests d'immuno-hématologie en phase solide et pourrait aussi trouver son application en phase liquide. Cette solution d'hématies indicatrices est stable pendant au moins 4 semaines, c'est-à-dire qu'on n'observe aucune désorption des antiglobulines.

Cette solution d'hématies est le premier réactif polyvalent mis au point. Son utilisation permet ainsi de réaliser le test de Coombs en phase solide, sur une seule microplaque, l'ensemble hématies-microplaque pouvant constituer un kit prêt à l'emploi.

Son utilisation permet de détecter à la fois les IgG fixant ou non le complément et les IgM fixant le complément, et, ainsi, de détecter toutes les classes d'anticorps irréguliers. L'utilisation de ce réactif est donc particulièrement adaptée aux tests de compatibilité sanguine préalables aux transfusions ainsi qu'au diagnostic de maladies hémolytiques et d'accidents transfusionnels. Ces utilisations sont également l'objet de la présente invention.

L'exemple suivant illustre l'invention sans en limiter la portée.

### Exemple

La mise en oeuvre de l'invention comprend plusieurs phases :
- la préparation du support (microplaque) ;
- la préparation des hématies indicatrices ;
- l'utilisation et la lecture du test.

L'exemple suivant s'applique plus particulièrement à la recherche des anticorps irréguliers, pour des tests de compatibilité sanguine.

### A. Préparation du support

On utilise comme support des microplaques à 96 puits du type utilisé couramment pour les cultures cellulaires.

Les hématies-tests phénotypées proviennent de donneurs de sang réguliers et bénévoles qui ont été phénotypés au minimum 2 fois dans la plupart des systèmes de groupes sanguins. Ces hématies sont suspendues dans une solution de conservation (de type Alsever) qui assure une stabilité de 28 jours.

Ces hématies-tests sont fixées au fond des puits des microplaques,
- soit par une liaison covalente, en déposant au fond des puits de la poly-L-lysine de poids moléculaire supérieur à 300 000 ou de la glutaraldéhyde,
- soit par une liaison immunologique, par le biais d'un anticorps spécifique (anti-I, anti-N, anti-glycophorine A...) d'un antigène présent sur les hématies-tests.

Les microplaques ainsi traitées sont stables au moins deux mois.

De la même manière, on peut fixer des anticorps au fond des puits.

### A.1. Exemple de traitement à la poly-L-lysine

Le support (la microplaque) est traité pendant une heure à 37°C à l'étuve avec une solution à 0,2 % en eau distillée de poly-L-lysine de poids moléculaire supérieur à 300 000 d. (d'après K. Carroll, B. Lannon and R. O'Kennedy. J. of Imm. Méthods 1990 ; 129, 71).

Le support est ensuite lavé 3 fois à l'eau distillée avant d'être conservé à l'abri de la lumière et de l'humidité. Ce support, ainsi traité, est stable au minimum 3 mois à température ambiante. Puis, il suffit d'ajouter 100 microlitres d'hématies-tests natives ou traitées par les enzymes protéolytiques, en suspension à 0,5 %. Le support est centrifugé 2 minutes à 120 g et lavé 3 fois en solution saline 0,15 M.

### A.2. Préparation de membranes d'hématies-tests

On peut remplacer les hématies par leurs membranes, préparées selon une technique décrite par Steck (Meth. of enzym; 1974 : 31; 172-180).

On utilise comme tampon d'hémolyse un tampon hypotonique au phosphate de sodium additionné de sulfate de magnésium et on laisse incuber les hématies pendant 10 minutes à température ambiante.

Les microplaques centrifugées à 120 g pendant 2 minutes avec les suspensions d'hématies à 0,5 % sont vidées par retournement, agitées pour décoller les "boutons" d'hématies en excès au fond des puits. Elles sont ensuite lavées 3 fois, doucement et jusqu'à disparition complète de la coloration rouge, avec le tampon, à l'aide d'une pipette à multicanaux (200 µl par puits à chaque lavage).

Les membranes fixées dans les puits et lysées in situ sont ensuite stabilisées dans une solution de glycérol à 40 % additionnée de 40 g/l de saccharose, de 1 mM de MgSO₄ et d'antibiotique. Les microplaques sont incubées 2 heures à + 4°C avec cette solution puis vidées, séchées, mises en sachet avec un dessiccateur et conservées à + 4°C.

Dans ces conditions, les membranes sont stables environ 7 semaines.

### B. Préparation des hématies indicatrices.

### B.1. Fixation du complément.

Un pool de 3 types d'hématies O Rh positif DCcEe est lavé 6 fois en eau physiologique puis 500 µl de culot globulaire lavé sont additionnés de 500 µl d'eau physiologique, dans un tube, puis de 8,5 ml de tampon phosphate saccharose et 300 µl d'un pool de sérum AB frais (comme source de complément).

### Préparation du tampon phosphate saccharose

1° solution K₂HPO₄ 1,0 M
   17,4 g/100 ml eau distillée solution KH₂PO₄ 1,0 M
   13,6 g/100 ml eau distillée
2°
   - solution A
      0,5 ml de K₂HPO₄ 1M/100 ml eau distillée
   - solution B
      0,5 ml de KH₂PO₄ 1M/100 ml eau distillée
3° tampon phosphate saccharose
   Additionner A dans B jusqu'à obtention du pH 6,1 (tampon phosphate)
   Ajouter 9,24 g saccharose/100 ml de tampon phosphate
   Aliquoter par 5 ml et congeler à -20°C.

Cette solution de saccharose a pour effet d'entraîner la fixation et l'activation directe du complément sur les hématies par la voie alterne.

Après incubation du tube au bain-marie à 37°C pendant 30 minutes, l'activation du complément en est au stade C₃bi. La suspension des hématies sensibilisées par le complément est centrifugée puis le culot globulaire est lavé à nouveau 4 fois jusqu'à disparition de l'hémolyse. Puis 1 ml de trypsine à 0,1 % est ajouté et le tube est à nouveau incubé au bain-marie à 37°C pendant 30 minutes ; la trypsine entraîne l'activation et le clivage du C₃bi en C₃d.

Les hématies O Rh positif recouvertes désormais de C₃d sont de nouveau lavées 4 fois en eau physiologique (jusqu'à disparition de l'hémolyse).

### B.2. Fixation d'immunoglobuline monoclonale IgG

A 150 µl de culot d'hématies recouvertes de C₃d sont ajoutés 200 µl d'immunoglobuline monoclonale IgG anti-D et 1 ml d'eau physiologique, dans un tube.

Celui-ci est incubé au bain-marie à 37°C pendant 30 minutes pour permettre la fixation de l'IgG anti-D. Puis les hématies sensibilisées sont à nouveau lavées 4 fois en eau physiologique.

### B.3 Fixation des antiglobulines anti-IgG et anti-C₃d

Au culot d'hématies O Rh positif recouvert de C₃d et d'anti-D sont ajoutés 300 µl d'antiglobuline anti-IgG et 1 ml d'antiglobuline anti-C₃d. Une dernière incubation est effectuée à température ambiante et sous agitation modérée mais permanente de façon à empêcher l'agglutination des hématies. Après 4 nouveaux lavages en eau physiologique, le culot d'hématies revêtu des 2 antiglobulines est suspendu en solution de conservation dans des tubes de verre stérile. Des essais de stabilité ont été réalisés : ces globules rouges ayant fixé l'anti-IgG et l'anti-C₃d sont utilisables pendant 4 semaines sans qu'une désorption importante des antiglobulines soit observée.

La difficulté de cette préparation réside dans l'équilibre des concentrations en hématies, en anticorps, en complément et en antiglobuline pour qu'il y ait sensibilisation des hématies sans qu'il y ait agglutination.

On utilise environ 100 µg/ml de l'anti-D IgG monoclonal (souche HM-16), 2,6 mg/ml d'antiglobuline anti-IgG et 2,7 mg/ml de protéines totales d'antiglobuline anti-C3d (d'activité spécifique environ égale à 30 %).

L'anticorps IgG anti-D utilisé est un anticorps monoclonal dont la lignée productrice a été développée par la Demanderesse, mais d'autres anticorps, de préférence monoclonaux, auraient aussi pu être utilisés.

### C. Utilisation et lecture du test

Les sérums à tester sont distribués dans les microplaques portant les cellules phénotypées (ou leurs membranes) ou des anticorps choisis.

On utilise généralement 50 microlitres de sérum par puits, éventuellement additionnés de 10 microlitres de "tampon de basse force ionique" (BFI, comme dans les tests classiques en tubes).

Les microplaques sont incubées 35 min à 37°C (ou 15 min en présence de BFI) puis elles sont lavées 3 fois en solution saline 0,15 M.

En cas de groupage sanguin ABO-D ou de phénotypage Rh-K, ce sont les hématies à tester qui sont ajoutées sur un complexe spécifique antigène-anticorps fixé au fond des puits (ex. hématies-tests D positif sensibilisées faiblement par un anti-D : si l'hématie à tester est D positive, elle réagira avec le complexe fixé au fond du puits).

Pendant l'incubation, les anticorps présents se fixent sur les antigènes correspondants et, éventuellement, entraînent l'activation du complément.

Cette réaction antigène-anticorps est visualisée indirectement par l'addition des hématies indicatrices décrites plus haut.

Ce test permet de détecter l'ensemble des anticorps irréguliers :
- IgG ne fixant pas le complément,
- IgG fixant le complément (lorsqu'il sont en très faible quantité, ils sont parfois détectés uniquement par l'activation du complément et la fixation de l'anti-complément, anti-C₃d),
- IgM fixant le complément.

A ce jour, 11 205 sérums de patients, 417 anticorps et 24 spécificités ont été étudiés.

Le tableau suivant montre la plus grande sensibilité du test réalisé en phase solide en comparaison avec le test classique en tube en présence également d'anti-C₃d et d'anti-IgG.

| | | Nombre d'anticorps détectés | |
|---|---|---|---|
| SPEFICITES ANTI- | TOTAL | en PHASE SOLIDE | en TUBE |
| C^{W} | 6 | 6 | 5 |
| D | 112 | 112 | 98 |
| C | 1 | 1 | 1 |
| E | 40 | 40 | 29 |
| C | 25 | 25 | 22 |
| e | 1 | 1 | 0 |
| D+C | 21 | 21 | 20 |
| D+C+E | 1 | 1 | 1 |
| c+E | 5 | 5 | 5 |
| K | 66 | 66 | 59 |
| Kpa | 2 | 2 | 2 |
| Fya | 14 | 14 | 13 |
| Jka | 6 | 6 | 6 |
| Jkb | 3 | 3 | 3 |
| M | 5 | 5 | 3 |
| Lua | 1 | 1 | 1 |
| S | 2 | 2 | 1 |
| s | 1 | 1 | 1 |
| Lea | 76 | 76 | 68 |
| Leb | 14 | 14 | 13 |
| Lea+Leb | 1 | 1 | 1 |
| E+K+Fya | 1 | 1 | 1 |
| E+K | 1 | 1 | 1 |
| c+K | 1 | 1 | 1 |
| c+S | 1 | 1 | 1 |
| Tja | 1 | 1 | 1 |
| P1 | 1 | 1 | 1 |
| Wra | 1 | 1 | 1 |
| Vel | 4 | 4 | 4 |
| Kna | 1 | 1 | 0 |
| Luke | 1 | 1 | 1 |
| Yta | 1 | 1 | 1 |

## Revendications

1. Hématies indicatrices prêtes à l'emploi pour la détection en immuno-hématologie, en phase solide, de complexes antigènes-anticorps, caractérisées en ce qu'elles portent à la fois de l'antiglobuline anti-IgG et de l'antiglobuline anti-C₃d.

2. Méthode de préparation des hématies indicatrices selon la revendication 1, caractérisée en ce qu'elle comprend les 3 étapes suivantes :
- une première étape de fixation du complément et d'activation de son fragment C₃d pour donner des hématies sensibilisées au C₃d,
- une deuxième étape de fixation d'immunoglobuline IgG dressée contre un marqueur antigénique des hématies indicatrices pour donner des hématies sensibilisées aux IgG,
- une troisième étape de fixation par immuno-adhérence d'antiglobuline anti-C₃d et d'antiglobuline anti-IgG sur les hématies sensibilisées.

3. Méthode selon la revendication 2, caractérisée en ce que la fixation du complément est réalisée en présence de saccharose.

4. Méthode selon la revendication 2 ou 3, caractérisée en ce que l'activation du fragment C₃d est obtenue par un traitement à la trypsine.

5. Méthode selon la revendication 2, caractérisée en ce que l'immunoglobuline est une IgG monoclonale anti-D.

6. Utilisation des hématies selon la revendication 1, pour la détection d'IgG fixant ou non le complément et d'IgM fixant le complément.

7. Utilisation selon la revendication 6 pour les tests de compatibilité sanguine préalables aux transfusions ou à but diagnostique.

8. Kit d'immunohématologie pour l'utilisation selon la revendication 6 ou 7, caractérisé en ce qu'il comprend des hématies selon la revendication 1, susceptibles d'être préparées par la méthode selon l'une quelconque des revendications 2 à 5.

## Patentansprüche

1. Anzeigende Erythrozyten, die für eine Verwendung in der Immuno-Hämatologie zum Nachweis von Antigen-Antikörper-Komplexen in fester Phase fertig sind, dadurch gekennzeichnet, daß sie gleichzeitig das Antiglobulin anti-IgG und das Antiglobulin anti-C₃d tragen.

2. Verfahren zur Herstellung von anzeigenden Erythrozyten nach Anspruch 1, dadurch gekennzeichnet, daß es die folgenden drei Schritte umfaßt:
- einen ersten Schritt der Fixierung von Komplement und der Aktivierung des Fragments C₃d, um C₃d-sensibilisierte Erythrozyten zu ergeben,
- einen zweiten Schritt einer Fixierung von Immunglobulin IgG, gerichtet gegen einen Antigen-Marker anzeigender Erythrozyten, um IgG-sensibilisierte Erythrozyten zu ergeben,
- einen dritten Schritt der Fixierung durch Immuno-Adhärenz von Antiglobulin anti-C₃d und Antiglobulin anti-IgG an die sensibilisierten Erythrozyten.

3. Verfahren nach Anspruch 2, dadurch gekennzeichnet, daß die Fixierung des Komplements in Gegenwart von Saccharose bewirkt wird.

4. Verfahren nach Anspruch 2 oder 3, dadurch gekennzeichnet, daß die Aktivierung des Fragments C₃d durch eine Behandlung mit Trypsin bewirkt wird.

5. Verfahren nach Anspruch 2, dadurch gekennzeichnet, daß das Immunglobulin ein monoklonales anti-D IgG ist.

6. Verwendung der Erythrozyten nach Anspruch 1 für den Nachweis von IgG, das Komplement bindet oder nicht und von IgM, das Komplement bindet.

7. Verwendung nach Anspruch 6 für Tests auf die Blutverträglichkeit vor Transfusionen oder zu einem diagnostischen Zweck.

8. Immuno-hämatologisches Kit für eine Verwendung nach Anspruch 6 oder 7, dadurch gekennzeichnet, daß es die Erythrozyten nach Anspruch 1 umfaßt, die durch das Verfahren nach einem oder mehreren der Ansprüche 2 bis 5 hergestellt werden können.

## Claims

1. Indicator erythrocytes ready to use for the immuno-haematologic detection, in solid phase, of antigen-antibody complexes, characterized in that they bear anti-IgG immunoglobulin together with anti-C₃d antiglobulin.

2. A process for preparing indicator erythrocytes according to claim 1, characterized in that it comprises the 3 following steps :
- a first step of binding the complement and activating its C₃d fragment to form C₃d sensitized erythrocytes,
- a second step of binding IgG immunoglobulin directed against an antigenic marker of the indicator erythrocytes to form IgG sensitized erythrocytes,
- a third step of immuno-adhesion binding of anti-C₃d antiglobulin and of anti-IgG antiglobulin onto the sensitized erythrocytes.

3. A process according to claim 2 characterized in that complement binding is performed in the presence of sucrose.

4. A process according to claim 2 or 3 characterized in that the activation of C₃d fragment is performed by trypsin treatment.

5. A process according to claim 2 characterized in that the immunoglobulin is an anti-D monoclonal IgG.

6. Use of erythrocytes according to claim 1 for the detection of IgG that bind or that do not bind complement and of IgM that bind complement.

7. Use according to claim 6 to perform blood compatibility tests before blood transfusion or for diagnostic purpose.

8. Immunohaematology kit for the use according to claim 6 or 7, characterized in that it comprises erythrocytes according to claim 1, which can be prepared by the process according to any of claims 2 to 5.
